# EUROPEAN PATENT APPLICATION

(11) **EP 4 813 464 A2**
(43) Date of publication of application: **30.09.2026**
(21) Application number: 26190371.0
(22) Date of filing: 24.01.2022
(51) Int. Cl.: A61P 43/00

(54) **PEPTIDE FOR PREVENTING AND TREATING FIBROSIS**

(30) Priority: 25.01.2021 KR 20210009981
(62) Document number(s) of the earlier application(s) in accordance with Art. 76 EPC: 22742932.1 / 4 282 874
(71) Applicant: Nibec Co., Ltd., Chungcheongbuk-do 27816 (KR)
(72) Inventor: CHUNG, Chong-Pyoung, 05618 Seoul (KR); PARK, Yoon Jeong, 08291 Seoul (KR); LEE, Jue-Yeon, 13831 Gwacheon-si Gyeonggi-do (KR); LEE, Dong Woo, 08652 Seoul (KR)
(74) Representative: Mewburn Ellis LLP

(57) **Abstract**

The present invention relates to a peptide for preventing or treating fibrosis, having the effects of preventing or treating fibrosis by having the property of inhibiting the dissociation of TGF-β1 from LCC by binding to an integrin or the property of inhibiting the change from epithelial cells to mesenchymal cells and promoting the change from mesenchymal cells to epithelial cells, and the property of penetrating cells to bind to Smad2 and Smad3, and thus suppresses the phosphorylation thereof. A peptide according to the present invention exhibits better therapeutic effects than Nintedanib, which was commercialized as a therapeutic agent for pulmonary fibrosis, and Liraglutide, which was commercialized as a therapeutic agent for hepatic fibrosis, and thus can be used as an alternative therapeutic agent to these commercially available therapeutic agents or as an agent for combination therapy with these therapeutic agents.

## Description

### Technical Field

The present invention relates to a peptide for preventing or treating fibrosis, and more particularly to a peptide for preventing or treating fibrosis, which inhibits the dissociation of TGF-β1 from LCC by binding to integrin, or which inhibits the transition of epithelial cells to mesenchymal cells and promotes the transition of mesenchymal cells to epithelial cells, and also binds to Smad2 and Smad3 through cell penetration to thus inhibit phosphorylation thereof, thereby exhibiting an effect of preventing or treating fibrosis.

### Background Art

Fibrosis is caused by excessive production and accumulation of collagen fibers in tissues, and is known to mainly occur in the lungs, liver, heart, kidneys, etc. As pulmonary fibrosis progresses, not only respiratory disorders but also various complications appear. Systemic hypoxia caused by reduced lung capacity and impaired oxygen diffusion, pulmonary hypertension caused by compression of pulmonary arteries and capillaries due to contraction of fibrous tissue, heart failure caused by blood pooling in the right ventricle due to impaired pulmonary circulation, and other complications such as thrombosis, pneumonia, and the like may occur. Also, inflammation and fibrosis may increase the risk of lung cancer. Pulmonary fibrosis may be caused by inhalation of toxic substances or continuous inflammation, but there is also idiopathic pulmonary fibrosis (IPF) for which a specific cause cannot be identified. When idiopathic pulmonary fibrosis (IPF) develops, lung tissue thickens and hardens over time, such that the lungs are unable to properly absorb oxygen and deliver the same to the bloodstream, ultimately incurring permanent scarring and fibrosis of the lungs, making breathing difficult, and reducing the amount of oxygen delivered to the body's major organs (Coward WR, The pathogenesis of idiopathic pulmonary fibrosis. Ther. Adv. Respir. Dis. 2010; 4:367-388).

Pulmonary fibrosis is a fatal disease with an average survival time of about 2 to 3 years after diagnosis, and there is still no way to restore lung tissue with advanced pulmonary fibrosis. Currently, steroids and immunosuppressants that suppress the inflammatory response are used as therapeutic agents for pulmonary fibrosis, but fibrosis progresses after the inflammatory response, and most patients visit hospitals with dyspnea, so treatment starts after the inflammatory response period has already passed and the fibrosis response period has begun, and therefore, it is difficult to expect a fibrosis inhibitory effect from existing anti-inflammatory drugs.

Meanwhile, among liver diseases, nonalcoholic steatohepatitis (NASH or nonalcoholic fatty liver disease (NAFLD)) is steatohepatitis caused by non-drinking obesity, diabetes, hyperlipidemia, drugs, etc. Nonalcoholic steatohepatitis develops into cirrhosis and liver cancer due to inflammation and hepatic fibrosis caused by hepatocellular degeneration/necrosis resulting from fat accumulation in hepatocytes. Therefore, even in case of nonalcoholic steatohepatitis, if fibrosis occurs, restoration to normal tissue is impossible. In addition to the lungs and liver, fibrosis may develop in the heart, kidneys, blood vessels, skin, brain, and the like.

One of currently available fibrosis drugs is nintedanib (product name: Ofev^{®}), which is a tyrosine kinase inhibitor that targets growth factor receptors involved in the mechanism of pulmonary fibrosis, among which platelet-derived growth factor receptor (PDGFR), fibroblast growth factor receptor (FGFR), and vascular endothelial growth factor receptor (VEGFR) are blocked (Wollin L, Mode of action of nintedanib in the treatment of idiopathic pulmonary fibrosis. Eur. Respir. J. 2015; 45:1434-1445; EP 1224170 B9). In addition, pirfenidone is used for the treatment of idiopathic pulmonary fibrosis, in which fibrosis progresses in the lungs without a clear cause. This drug is a small molecule oral agent that inhibits the synthesis of collagen, reduces profibrotic cytokines, and suppresses the proliferation of fibroblasts that produce collagen (US 8383150 B2).

In a normal state, collagen in tissues is kept constant, but in some pathological conditions, the balance is broken, and collagen is excessively produced and accumulated in tissues, leading to fibrosis. Inhibitors that decrease fibrosis mainly perform the functions of 1) reducing the inflammatory response related to fibrosis, 2) inhibiting the activity of profibrotic cytokines or growth factors, 3) suppressing cell proliferation, and 4) reducing procollagen biosynthesis and processing, and therefore, these mechanisms of action are major targets of fibrosis inhibitors. TGF-β (transforming growth factor-beta) is known as a representative profibrotic growth factor, and a compound that directly inhibits TGF-β is being used to develop a fibrosis therapeutic agent. However, since TGF-β is involved in various physiological responses *in vivo*, direct inhibition by targeting TGF-β or the TGF-β receptor may have great side effects. Hence, a drug with a mechanism that indirectly inhibits the action of TGF-β is needed.

When looking at fibrosis from a cellular point of view, when normal epithelial cells are damaged by inflammation or some stimulus, the expression of TGF-β increases and the transition of epithelial cells to mesenchymal cells (epithelial-mesenchymal transition, EMT) increases, resulting in transition of epithelial cells to myofibroblasts. It is known that such myofibroblasts ultimately cause fibrosis by excessively producing extracellular matrix proteins such as collagen (Rock JR, Multiple stromal populations contribute to pulmonary fibrosis without evidence for epithelial to mesenchymal transition. Proc. Natl. Acad. Sci. USA 2011; 108:E1475-E1483; Fernandez IE, The impact of TGF-b on lung fibrosis: from targeting to biomarkers. Proc. Am. Thorac. Soc. 2012; 9:111-116; Decaris ML, Proteomic analysis of altered extracellular matrix turnover in bleomycin-induced pulmonary fibrosis. Mol. Cell Proteomics 2014; 13:1741-1752). Therefore, excessive transition of epithelial cells to myofibroblasts is prevented, and on the contrary, transition of mesenchymal cells to normal epithelial cells (mesenchymal-epithelial transition, MET), namely cell reprogramming, is promoted to thus treat fibrosis.

Accordingly, the present inventors have ascertained that the peptide developed by the present inventors does not directly inhibit TGF-β1, but binds to integrin present in lung fibroblasts to thus prevent TGF-β1 from dissociating from LCC (large latent complex), so fibrosis is suppressed by inhibiting TGF-β1-induced profibrotic signaling, and also that the peptide inhibits transition of epithelial cells into mesenchymal cells and simultaneously promotes transition of mesenchymal cells to epithelial cells, inducing cell reprogramming, and binds to Smad2 and Smad3 through cell penetration to thus inhibit phosphorylation thereof, thereby suppressing fibrosis, and furthermore, the peptide is effective at reducing fibrosis when administered to an animal model of pulmonary fibrosis or an animal model of hepatic fibrosis induced by nonalcoholic steatohepatitis, thereby culminating in the present invention.

The information described in the Background Art is only for improving understanding of the background of the present invention, and it is not to be construed as including information forming the related art already known to those of ordinary skill in the art to which the present invention belongs.

### Summary of the Invention

It is an object of the present invention to provide a peptide that is effective at preventing and treating fibrosis.

It is another object of the present invention to provide a pharmaceutical composition for preventing or treating fibrosis comprising the peptide.

It is still another object of the present invention to provide a method of preventing or treating fibrosis comprising administering the peptide.

It is yet another object of the present invention to provide the use of the peptide for preventing or treating fibrosis.

It is still yet another object of the present invention to provide the use of the peptide for the manufacture of a medicament for preventing or treating fibrosis.

In order to accomplish the above objects, the present invention provides a peptide for preventing or treating fibrosis represented by the amino acid sequence of SEQ ID NO: 1 or SEQ ID NO: 2.

In addition, the present invention provides a pharmaceutical composition for preventing or treating fibrosis comprising the peptide represented by the amino acid sequence of SEQ ID NO: 1 and/or the peptide represented by the amino acid sequence of SEQ ID NO: 2 as an active ingredient.

In addition, the present invention provides a method of preventing or treating fibrosis comprising administering the peptide represented by the amino acid sequence of SEQ ID NO: 1 or SEQ ID NO: 2 or the composition comprising the peptide to a subject in need thereof.

In addition, the present invention provides the use of the peptide represented by the amino acid sequence of SEQ ID NO: 1 or SEQ ID NO: 2 or the composition comprising the peptide for preventing or treating fibrosis.

In addition, the present invention provides the use of the peptide represented by the amino acid sequence of SEQ ID NO: 1 or SEQ ID NO: 2 or the composition comprising the peptide for the manufacture of a medicament for preventing or treating fibrosis.

### Brief Description of Drawings

FIG. 1 schematically shows an effect of preventing or treating fibrosis by inhibiting TGF-β1-induced profibrotic intracellular signaling using a peptide according to the present invention that binds to integrin.
FIG. 2a schematically shows an effect of preventing or treating fibrosis by inhibiting the transition of epithelial cells to mesenchymal cells and promoting the transition of mesenchymal cells to epithelial cells using a peptide according to the present invention.
FIG. 2b schematically shows an effect of preventing or treating fibrosis by inhibiting profibrotic intracellular signaling using the peptide according to the present invention that binds to Smad2 and Smad3 through cell penetration to thus inhibit phosphorylation thereof.
FIG. 3 shows results confirming the ability of the peptide of SEQ ID NO: 1 to bind to integrin β1, β3, αv, α4, and α5.
FIG. 4 shows results confirming the ability of the peptide of SEQ ID NO: 2 to bind to Smad2 or Smad3.
FIG. 5 shows results of inhibiting the expression of pSmad2 and pSmad3 by the peptide of SEQ ID NO: 2.
FIG. 6 shows results confirming the effect of the peptide of SEQ ID NO: 1 or 2 on inhibiting COL1A2 and COL3A1 gene expression.
FIG. 7a shows results of observing an increase in MET marker expression by the peptide of SEQ ID NO: 2.
FIG. 7b shows results of observing inhibition of EMT marker expression by the peptide of SEQ ID NO: 2.
FIG. 8a shows an experimental protocol for confirming the therapeutic effect upon treatment of a pulmonary fibrosis animal model with the peptide according to the present invention.
FIG. 8b shows results of observing a weight change upon treatment of a pulmonary fibrosis animal model with the peptide according to the present invention.
FIG. 8c shows results confirming an effect of reducing fibrous deposition upon treatment of a pulmonary fibrosis animal model with the peptide according to the present invention.
FIG. 8d shows results confirming the fibrosis reduction effect by measuring the area of fibrosis upon treatment of a pulmonary fibrosis animal model with the peptide according to the present invention.
FIG. 9a shows an experimental protocol for confirming the therapeutic effect upon treatment of a hepatic fibrosis animal model with the peptide according to the present invention.
FIG. 9b shows results of H&E staining to confirm the therapeutic effect upon treatment of a hepatic fibrosis animal model with the peptide according to the present invention.
FIG. 9c shows results of measuring the NAFLD activity score to confirm the therapeutic effect upon treatment of a hepatic fibrosis animal model with the peptide according to the present invention.
FIG. 9d shows results of Masson's trichrome staining to confirm the therapeutic effect upon treatment of a hepatic fibrosis animal model with the peptide according to the present invention.
FIG. 9e shows results of measuring the area of fibrosis to confirm the therapeutic effect upon treatment of a hepatic fibrosis animal model with the peptide according to the present invention.

### Detailed Description and Preferred Embodiments of the Invention

Unless otherwise defined, all technical and scientific terms used herein have the same meanings as typically understood by those skilled in the art to which the present invention belongs. In general, the nomenclature used herein and experimental methods described later are well known in the art and are typical.

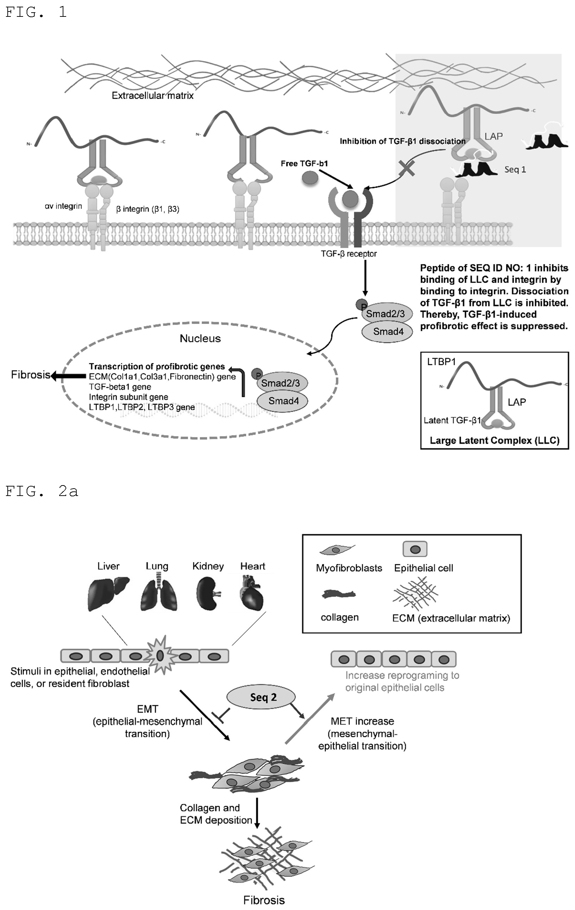

TGF-β1 is known as a protein that plays an important role in increasing fibrosis. *In vivo*, TGF-β1 binds to LLC (large latent complex) and exists in a latent form. LLC is composed of LAP (latency-associated peptide), LTBP1 (latent TGF-β-binding protein), and TGF-β1. When LAP in LLC binds to an integrin receptor on the cell surface, latent TGF-β1 is dissociated from LLC. This free-TGF-β1 binds to the TGFβ receptor, causing profibrotic intracellular signaling. The peptide of SEQ ID NO: 1 binds to integrin, thus inhibiting LLC from reacting with integrin, thereby preventing the release of TGF-β1. Ultimately, the expression of fibrosis-related proteins by TGF-β1 is suppressed (FIG. 1).

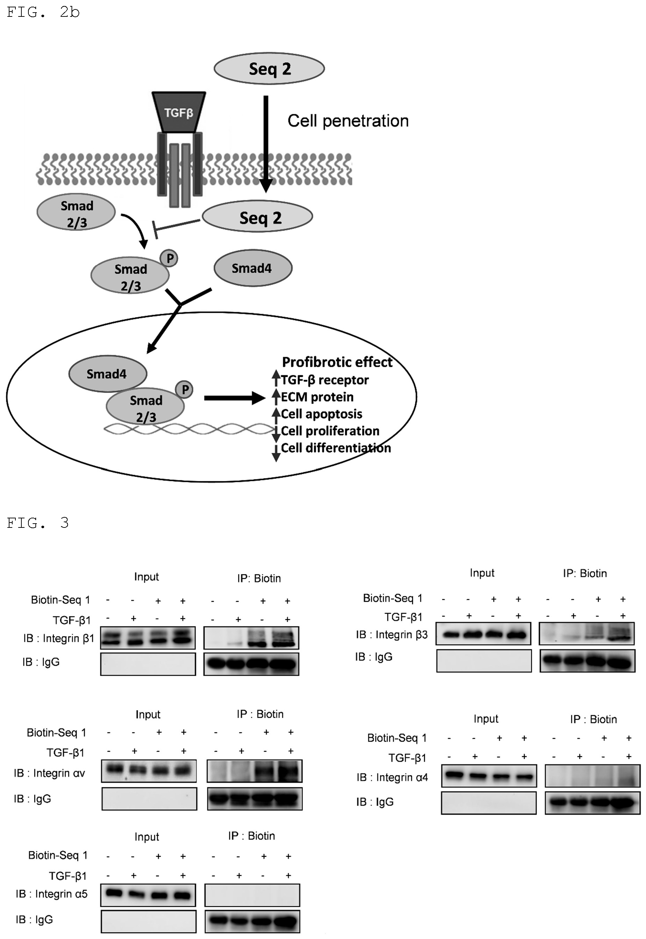

In fibrosis, normal epithelial cells are damaged, and the expression of TGF-β1 increases, so epithelial-mesenchymal transition (EMT) increases, and thus transition of epithelial cells to myofibroblasts occurs. It is known that such myofibroblasts ultimately induce fibrosis by excessively producing extracellular matrix proteins such as collagen. In the present invention, the peptide of SEQ ID NO: 2 inhibits excessive transition of epithelial cells to mesenchymal cells, particularly myofibroblasts, and conversely, promotes the transition of mesenchymal cells to normal epithelial cells (mesenchymal-epithelial transition, MET), that is, increases cell reprogramming, confirming that matrix protein deposition may be inhibited and fibrosis may be treated (FIG. 2a). In addition, the peptide of SEQ ID NO: 2 suppresses TGF-β1-induced profibrotic effects by inhibiting phosphorylation of Smad2 and Smad3 increased by TGF-β1. The peptide of SEQ ID NO: 2, which is capable of penetrating cells, binds to Smad2 and Smad3 proteins in cells to thus inhibit phosphorylation thereof, consequently suppressing TGF-β1-induced fibrosis (FIG. 2b).

Accordingly, an aspect of the present invention pertains to a peptide for preventing or treating fibrosis represented by the amino acid sequence of SEQ ID NO: 1 or the amino acid sequence of SEQ ID NO: 2 below.
SEQ ID NO: 1: YGLRSKSKKFRRPDIQYPDAT
SEQ ID NO: 2: GKCSTRGRKSSRRKK

In the present invention, the peptide represented by the amino acid sequence of SEQ ID NO: 1 prevents the dissociation of TGF-β1 from LLC (large latent complex) by binding to integrin, thus inhibiting TGF-β1-induced profibrotic intracellular signaling, thereby suppressing fibrosis.

In the present invention, the peptide represented by the amino acid sequence of SEQ ID NO: 2 inhibits the epithelial-mesenchymal transition, and also binds to Smad2 and Smad3 through cell penetration to thus inhibit phosphorylation thereof, thereby suppressing fibrosis.

Another aspect of the present invention pertains to a pharmaceutical composition for preventing or treating fibrosis comprising the peptide represented by the amino acid sequence of SEQ ID NO: 1 and/or the peptide represented by the amino acid sequence of SEQ ID NO: 2 as an active ingredient.

In the present invention, fibrosis is a disease caused by excessive deposition of collagen, and may be selected from the group consisting of pulmonary fibrosis, hepatic fibrosis, renal fibrosis, and cardiac fibrosis, but is not limited thereto, and the peptide and the pharmaceutical composition according to the present invention may be used for the prevention or treatment of all fibrosis diseases caused by excessive collagen deposition.

In the present invention, the pharmaceutical composition may be formulated into any one dosage form selected from the group consisting of an injection, a formulation for oral administration, a patch, a liquid, a capsule, a granule, a tablet, a powder, a spray, a formulation for nasal administration, an inhalation spray, an ointment, a gel, a formulation for mucosal administration, and a suppository, but is not limited thereto. These formulations may be prepared by a typical method used for formulation in the art or a method disclosed in Remington's Pharmaceutical Science (recent edition), Mack Publishing Company, Easton PA, and may be prepared in various forms depending on individual diseases or ingredients. However, the above description is illustrative, and formulations applicable to the present invention are not limited thereto.

In the present invention, the pharmaceutical composition may further comprise an acceptable adjuvant, and the adjuvant may be, for example, a carrier. A pharmaceutically acceptable carrier may be saline, sterile water, Ringer's solution, buffered saline, dextrose solution, maltodextrin solution, glycerol, ethanol, and a mixture of one or more thereof, and, as necessary, other typical additives such as antioxidants, buffers, bacteriostatic agents, etc. may be added. Also, diluents, dispersants, surfactants, binders, and lubricants may be further added to prepare injectable formulations such as aqueous solutions, suspensions, emulsions, etc., formulations for nasal administration, inhalation sprays, pills, capsules, granules, or tablets. However, the above description is illustrative, and the adjuvant or carrier usable in the present invention is not limited thereto.

The composition of the present invention may be administered orally or parenterally (e.g. intravenous, subcutaneous, intraperitoneal, intranasal, intratracheal, or topical application) depending on the desired method, and the dose thereof varies depending on the patient's body weight, age, gender, health status, diet, administration time, administration method, excretion rate, and severity of disease. The peptide of SEQ ID NO: 1 or 2 of the present invention may be administered daily at about 1 µg/kg to 100 mg/kg, preferably 5 µg/kg to 50 mg/kg, and may be administered every day or 1-3 times a week. However, the dose and administration interval are not limited thereto.

Here, the administration regimen and dose may vary depending on the age, body weight, and response of the individual patient. Appropriate administration regimens and doses may be readily selected by those skilled in the art who take these factors into account.

Still another aspect of the present invention pertains to a method of preventing or treating fibrosis comprising administering the peptide represented by the amino acid sequence of SEQ ID NO: 1 or the peptide represented by the amino acid sequence of SEQ ID NO: 2 to a subject in need thereof.

Yet another aspect of the present invention pertains to a method of preventing or treating fibrosis comprising administering the composition comprising the peptide represented by the amino acid sequence of SEQ ID NO: 1 and/or the peptide represented by the amino acid sequence of SEQ ID NO: 2 to a subject in need thereof.

A further aspect of the present invention pertains to the use of the peptide represented by the amino acid sequence of SEQ ID NO: 1 or the peptide represented by the amino acid sequence of SEQ ID NO: 2 for preventing or treating fibrosis.

Still a further aspect of the present invention pertains to the use of the composition comprising the peptide represented by the amino acid sequence of SEQ ID NO: 1 and/or the peptide represented by the amino acid sequence of SEQ ID NO: 2 for preventing or treating fibrosis.

Yet a further aspect of the present invention pertains to the use of the peptide represented by the amino acid sequence of SEQ ID NO: 1 or the peptide represented by the amino acid sequence of SEQ ID NO: 2 for the manufacture of a medicament for preventing or treating fibrosis.

Still yet a further aspect of the present invention pertains to the use of the composition comprising the peptide represented by the amino acid sequence of SEQ ID NO: 1 and/or the peptide represented by the amino acid sequence of SEQ ID NO: 2 for the manufacture of a medicament for preventing or treating fibrosis.

Since the above-described "peptide or composition comprising the peptide" is employed in the preventive or therapeutic method and use of the present invention, a redundant description thereof will be omitted.

In the present invention, a specific amino acid sequence of SEQ ID NO: 1 or SEQ ID NO: 2 is described, but it will be apparent to those skilled in the art that, within the scope of the technical idea of the present invention, an amino acid sequence of an equivalent range in which the amino acid sequence of the present invention is partially modified is construed as being within the scope of the present invention.

Hereinafter, the present invention will be described in more detail with reference to examples. These examples are only for illustrating the present invention, and it will be apparent to those skilled in the art that the scope of the present invention is not to be construed as being limited by these examples.

### Example 1: Peptide synthesis

Amino acids and reagents required for synthesis were purchased from GL Biochem and Sigma-Aldrich. Peptides were synthesized by an F-moc solid-phase chemical synthesis method from the C-terminus using a synthesizer. Specifically, a rink resin (0.075 mmol/g, 100-200 mesh, 1% DVB crosslinking) with Fmoc-(9-fluorenylmethoxycarbonyl) as a blocking group was used for synthesis, and 50 mg of the rink resin was placed in a synthesizer, the resin was swollen with DMF, and then a 20% piperidine/DMF solution was added to remove the Fmoc-group. A 0.5 M amino acid solution (solvent: dimethylformamide, DMF), 1.0 M DIPEA (solvent: dimethylformamide & N-methylpyrrolidone, DMF & NMP), and 0.5 M HBTU (solvent: dimethylformamide, DMF) were added in the sequence from the C-terminus in respective amounts of 5, 10, and 5 equivalent weights and allowed to react for 1 to 2 hours under a nitrogen stream. Whenever the deprotection and coupling steps were terminated, washing was performed twice with DMF and isopropanol. After coupling the last amino acid, deprotection was performed to remove the Fmoc-group.

The synthesis was confirmed using a ninhydrin test method, and the tested and synthesized resin was dried with tetrahydrofuran (THF) or dichloromethane (DCM), after which a trifluoroacetic acid (TFA) cleavage cocktail was added in an amount of 20 ml relative to 1 g of resin, shaken for 3 hours, and then filtered to separate the cocktail in which the resin and peptide were dissolved. After removing the filtered solution using a rotary evaporator, cold ether was added or an excess of cold ether was directly added to the TFA cocktail solution in which the peptide was dissolved, so the peptide was crystallized into a solid phase, which was then separated through centrifugation. Here, the TFA cocktail was completely removed through washing with ether several times and centrifugation. The peptide thus obtained was dissolved in distilled water and lyophilized.

The synthesized peptide was cleaved from the resin, washed, lyophilized, and separated and purified by high-performance liquid chromatography (Shimadzu, Japan). Analysis was performed in a manner in which 0.1% TFA/H₂O and 0.092% TFA/acetonitrile were allowed to flow for 30 minutes with a change of 0 to 60% at a flow rate of 1 ml/min using a C₁₈ column with a diameter of 4.6 mm. Here, the wavelength of a UV detector was set to 220 nm. Purification was carried out under the same solvent and detection wavelength conditions at a flow rate of 20 ml/min using a column with a diameter of 2.2 cm. The molecular weight of the purified peptide was determined through mass spectrometry.

The peptides of SEQ ID NO: 1 and SEQ ID NO: 2 below were synthesized in the same manner as above.
SEQ ID NO: 1: YGLRSKSKKFRRPDIQYPDAT
SEQ ID NO: 2: GKCSTRGRKSSRRKK

### Example 2: Confirmation of ability of peptide of SEQ ID NO: 1 to bind to integrin using immunoprecipitation

Human lung fibroblasts (Diseased human lung fibroblasts from IPF patients, DHLF-IPF, Lonza, #CC-7231) derived from patients with idiopathic pulmonary fibrosis were cultured in full growth media (Fibroblast Growth Basal Medium-2 supplemented with hFGF-B, Insulin, GA-1000, and 2% FBS, Lonza #CC-3132). Before treatment of 3x10⁶ DHLF-IPF with TGF-β1 (R&D systems, #240-B-002), the medium was replaced with Opti-MEM (Gibco, #31985-070), followed by culture for 24 hours. After 24 hours, the medium was replaced with existing growth media and the cells were treated with 10 ng/ml TGF-β1 (R&D systems, #240-B-002) for 24 hours. After 24 hours, the cells were treated for 1 hour with 100 µM biotin-bound peptide of SEQ ID NO: 1 (Biotin-Seq 1). The cells treated with 100 µM biotin-bound peptide of SEQ ID NO: 1 (Biotin-Seq 1) alone were also included in the group.

The cells thus treated were washed with ice-cold PBS, 500 µl of RIPA buffer (10 mM Tris-HCl, pH 8.0, 1 mM EDTA, 0.5 mM EGTA, 1% Triton X-100, 0.1% sodium deoxycholate, 0.1% SDS, 140 mM NaCl, complete protease inhibitor cocktail (Sigma-Aldrich), phosphatase inhibitor cocktail (Sigma-Aldrich)) was added thereto, and the cells were lysed for 20 minutes. Thereafter, centrifugation was performed at 13,000 g for 25 minutes, and cell lysates were allowed to react with an anti-biotin antibody (diluted 1:100; Abcam, ab53494) overnight at 4°C using a vertical rocker. 20 µl of protein-A agarose beads (Santa Cruz Biotechnology, sc-2003) was added thereto, followed by additional reaction for 3 hours. Centrifugation was briefly performed at 13,000 g, the supernatant was discarded, and the beads were washed with PBS. Protein bound to the beads was separated by adding 5x SDS sample buffer and then boiling the beads, followed by SDS-PAGE and Western blotting. Immunoblotting was performed using antibodies against integrin β1, β3, αv, α4, α5 (Santa Cruz Biotechnology, sc-374429, sc-46655, sc-9969, sc-365209, sc-376199), and IgG (Cell Signaling Technology). Protein levels were represented as whole lysate (input) and immunoprecipitated protein (IP).

As a result, the peptide of SEQ ID NO: 1 (Biotin-Seq 1) had the ability to selectively bind to integrin β1, β3, and αv, and upon simultaneous treatment with the peptide of SEQ ID NO: 1 and TGF-β1, the ability to bind to integrin β1, β3, and αv was further increased (FIG. 3). This means that the ability of the peptide of SEQ ID NO: 1 to selectively bind to integrin β1, β3, and αv is further increased in pathological conditions where TGF-β1 is high.

### Example 3: Confirmation of ability of peptide of SEQ ID NO: 2 to bind to Smad2 and Smad3 using immunoprecipitation

LX-2 (Human hepatic stellate cell line, Millipore #SCC064) was cultured in medium containing 5% DMEM High glucose (Gibco, 11965092), 10% FBS (Gibco, 16000044), and 1% antibiotics-antimycotic (Gibco, 15240062).

3x10⁶ LX-2 cells were treated with 100 µM biotin-bound peptide of SEQ ID NO: 2 (Biotin-Seq 2) for 1 hour. The cells thus treated were washed with ice-cold PBS, 500 µl of RIPA buffer (10 mM Tris-HCl, pH 8.0, 1 mM EDTA, 0.5 mM EGTA, 1% Triton X-100, 0.1% sodium deoxycholate, 0.1% SDS, 140 mM NaCl, complete protease inhibitor cocktail (Sigma-Aldrich), phosphatase inhibitor cocktail (Sigma-Aldrich)) was added thereto, and the cells were lysed for 20 minutes. Thereafter, centrifugation was performed at 13,000 g for 25 minutes, and cell lysates were allowed to react with an anti-biotin antibody (diluted 1:100; OriGene) overnight at 4°C using a vertical rocker. 20 µl of protein-A agarose beads (Santa Cruz Biotechnology, sc-2003) was added thereto, followed by additional reaction for 3 hours. Centrifugation was briefly performed at 13,000 g, the supernatant was discarded, and the beads were washed with PBS. Protein bound to the beads was separated by adding 5x SDS sample buffer and then boiling the beads, followed by SDS-PAGE and Western blotting. Immunoblotting was performed using antibodies against Smad2 (Cell Signaling, #5339) and Smad3 (Cell Signaling, #9513). Protein levels were represented as whole lysate (input) and immunoprecipitated protein (IP).

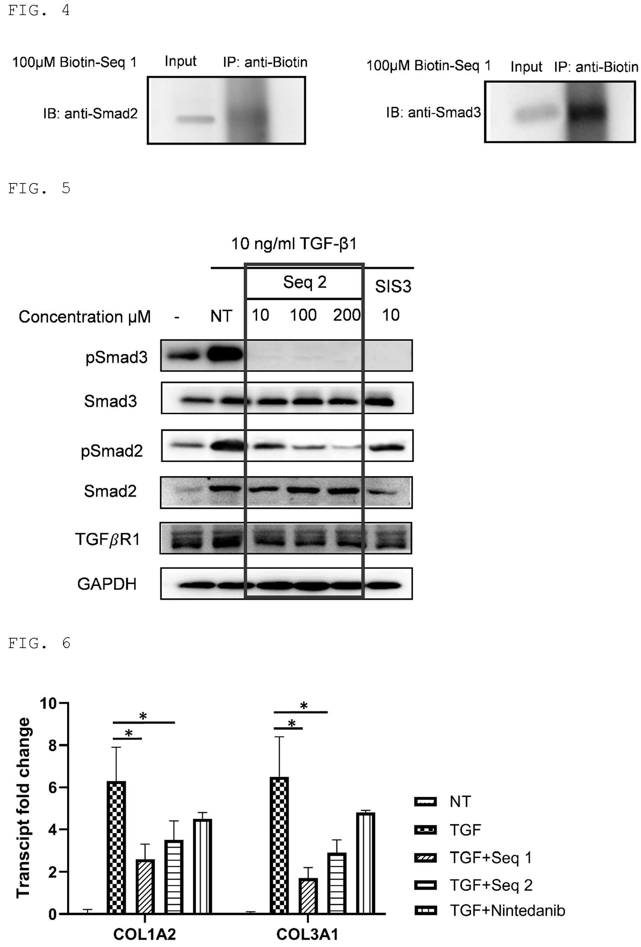

As a result, the peptide of SEQ ID NO: 2 (Biotin-Seq 2) showed the ability to bind to Smad2 and Smad3, particularly higher ability to bind to Smad3 (FIG. 4), indicating that the peptide of SEQ ID NO: 2 acts on Smad2 and Smad3, which are sub-signals of TGF-β1.

### Example 4: Confirmation of inhibition of phosphorylation of Smad2 and Smad3 by peptide of SEQ ID NO: 2

LX-2 (Human hepatic stellate cell line, Millipore #SCC064) was cultured in medium containing 5% DMEM High glucose (Gibco, 11965092), 10% FBS (Gibco, 16000044), and 1% antibiotics-antimycotic (Gibco, 15240062). 3x10⁶ LX-2 cells were treated with 10 ng/ml TGF-β1 and cultured for 24 hours. The cells were treated with 10, 100, or 200 µM of the peptide of SEQ ID NO: 2 or 10 µM SIS3 (Smad3 inhibitor, Sigma-Aldrich, catalog #566405) in fresh medium and cultured for 24 hours.

Proteins were lysed using RIPA lysis buffer (25 mM Tris HCl pH 7.6, 150 mM NaCl, 1% NP-40, 1% sodium deoxycholate, 0.1% SDS) containing a protease inhibitor and a phosphatase inhibitor. Proteins were quantified by BCA protein assay, and the expression levels of pSmad2, Smad2, pSmad3, Smad3, TGFβ receptor 1, and GAPDH proteins were determined by Western blotting. For Western blotting, the sample was loaded in the same amount along with a size marker on an 8% SDS PAGE gel, electrophoresed for about 2 hours, and then transferred to a nitrocellulose membrane. The transferred membrane was blocked with 5% skim milk for 1 hour, and allowed to react with primary antibodies at a ratio of 1:1000 overnight. The primary antibodies used were pSmad2 (Cell Signaling, #3108), Smad2 (Cell Signaling, #5339), pSmad3 (Abcam, ab52903), Smad3 (Cell Signaling, #9513), TGFβ receptor1 (Abcam, ab31013), and GAPDH (Cell Signaling, #2118S). Thereafter, the membrane was washed with TBST containing 0.1% Tween 20, and allowed to react with HRP-conjugated secondary antibodies anti-rabbit (Bethyl, A120-101P) and anti-mouse (Bethyl, A90-116P) at a ratio of 1:5000 for 1 hour, followed by chemiluminescence with an ECL substrate.

As a result, the expression of pSmad3 completely disappeared from the concentration of the peptide of SEQ ID NO: 2 of 10 µM, and the expression of pSmad2 decreased with an increase in the concentration of the peptide of SEQ ID NO: 2 (FIG. 5). However, there was no change in the expression level of the TGFβ receptor. This means that the peptide of SEQ ID NO: 2 binds to Smad2 and Smad3 to thus inhibit phosphorylation thereof.

### Example 5: Confirmation of inhibition of collagen gene expression in cells

Human lung fibroblasts (Lonza, #CC-7231) derived from patients with idiopathic pulmonary fibrosis were cultured in full growth media (Fibroblast Growth Basal Medium-2 supplemented with hFGF-B, Insulin, GA-1000, and 2% FBS, Lonza #CC-3132). The cells were seeded in a 24-well plate and cultured in starvation medium (Dulbecco's modified Eagle medium/F12 without FBS supplemented with 0.1 mM 2-phospho-L-ascorbic acid) for 24 hours. Recombinant Human TGF-β1 Protein (R&D Systems, #240-B-002) was diluted at 20 µg/ml in sterile 4 mM HCl containing 1 mg/ml bovine serum albumin. The cells were cultured for 24 hours in full growth media containing 10 ng/ml TGF-β1. The cells were treated with SEQ ID NO: 1 (200 µM), SEQ ID NO: 2 (200 µM), or nintedanib (10 µM) in fresh medium and cultured for 24 hours. The peptides were dissolved in purified water, nintedanib was dissolved in dimethyl sulfoxide, and the concentration of dimethyl sulfoxide in medium was set to 1%. The medium was removed, followed by washing with DPBS (Welgene, LB001-02) and cell lysis using 1 ml of a trizol reagent (Ambion, 15596). 250 µl of chloroform was added thereto, the tube was stirred, and centrifugation was performed at 10,000 rpm for 15 minutes. After removing the aqueous layer using a pipette, 550 µl of isopropanol (Sigma, I9516) was added and shaken gently. After centrifugation at 15,000 rpm for 25 minutes, isopropanol was removed, and 1 ml of 75% ethanol was added thereto. After removal of ethanol and drying in air, the pellets were dissolved in DEPC water (Invitrogen, AM9915G). The concentration of RNA was measured using a Nano Drop (Thermo Scientific, NanoDrop One, AZY1913798).

cDNA was synthesized from the extracted RNA using a Maxima First strand cDNA synthesis kit (Thermo Fisher, K1641) according to the manufacturer's protocol. In addition, real-time PCR was performed using QuantStudio 3 (Applied Biosystems, A28132) according to the manufacturer's protocol. PCR amplification was conducted by repeating 50 cycles at 95°C for 30 seconds, 60°C for 30 seconds, and 72°C for 30 seconds. Here, the primer sequences used are shown in Table 1 below.

**[Table 1] Primer sequence**

| Gene | Primer sequence(5' -> 3') | | SEQ ID NO. |
|---|---|---|---|
| Human-COL1a1 | Forward | CCTGGAAAGAATGGAGATGATG | 3 |
| | Reverse | ATCCAAACCACTGAAACCTCTG | 4 |
| Human-COL3a1 | Forward | GCTGGCTACTTCTCGCTCTG | 5 |
| | Reverse | TCCGCATAGGACTGACCAAG | 6 |
| Human-GAPDH | Forward | GGTGAAGGTCGGTGTGAACG | 7 |
| | Reverse | CTCGCTCCTGGAAGATGGTG | 8 |

As a result, treatment with TGF-β1 increased COL1A2 and COL3A1 gene expression, but when the cells were treated with TGF-β1 and then the peptide of SEQ ID NO: 1 or SEQ ID NO: 2, the expression of each collagen gene was significantly reduced (*p<0.05), confirming that the peptide of the present invention was more effective than nintedanib (FIG. 6).

### Example 6: Confirmation of reprogramming effects in cells

LX-2 (Human hepatic stellate cell line, Millipore #SCC064) was seeded at 1x10⁶ in each well of a 6-well plate (Nunc, 140675), and cultured in medium containing 5% DMEM High glucose (Gibco, 11965092), 10% FBS (Gibco, 16000044), and 1% antibiotics-antimycotic (Gibco, 15240062). After starvation in Opti-MEM^{™} (Gibco, 31985-070) for 6 hours, the medium was replaced with fresh Opti-MEM^{™}, and the cells were treated with 10 ng/ml TGF-beta1 and 100 µM of the peptide of SEQ ID NO: 2 for 24 hours. The medium was removed, followed by washing with DPBS (Welgene, LB001-02) and cell lysis using 1 ml of a trizol reagent (Ambion, 15596). 250 µl of chloroform was added thereto, the tube was stirred, and centrifugation was performed at 10,000 rpm for 15 minutes. After removing the aqueous layer using a pipette, 550 µl of isopropanol (Sigma, I9516) was added and shaken gently. After centrifugation at 15,000 rpm for 25 minutes, isopropanol was removed, and 1 ml of 75% ethanol was added thereto. After removal of ethanol and drying in air, the pellets were dissolved in DEPC water (Invitrogen, AM9915G). The concentration of RNA was measured using a Nano Drop (Thermo Scientific, NanoDrop One, AZY1913798).

cDNA was synthesized from the extracted RNA using a Maxima First strand cDNA synthesis kit (Thermo Fisher, K1641) according to the manufacturer's protocol. In addition, real-time PCR was performed using QuantStudio 3 (Applied Biosystems, A28132) according to the manufacturer's protocol. PCR amplification was conducted by repeating 40 cycles at 95°C for 15 seconds, 60°C for 50 seconds, and 72°C for 15 seconds. Here, the primer sequences used are shown in Table 2 below.

**[Table 2] Primer sequence**

| Gene | Primer Sequence(5'->3') | | SEQ ID NO. |
|---|---|---|---|
| E-cad | Forward | AGGGGTTAAGCACAACAGCA | 9 |
| | Reverse | ACGACGTTAGCCCGTTCTC | 10 |
| EpCAM | Forward | CGCAGCTCAGGAAGAATGTG | 11 |
| | Reverse | TGAAGTACACTGGCATGACG | 12 |
| N-cad | Forward | TGGGAATCCGACGAATGG | 13 |
| | Reverse | TGCAGATCGGACCGGATACT | 14 |
| Twist | Forward | TGTCCATTTTCTCCTTCTCTGGA | 15 |
| | Reverse | TGTCCGCGTCCCACTAGC | 16 |
| Snail | Forward | GGCCTAGCGAGTGGTTCTTC | 17 |
| | Reverse | GTTAGGCTTCCGATTGGGGT | 18 |
| GAPDH | Forward | ACATCGCTCAGACACCATG | 19 |
| | Reverse | TGTAGTTGAGGTCAATGAAGGG | 20 |

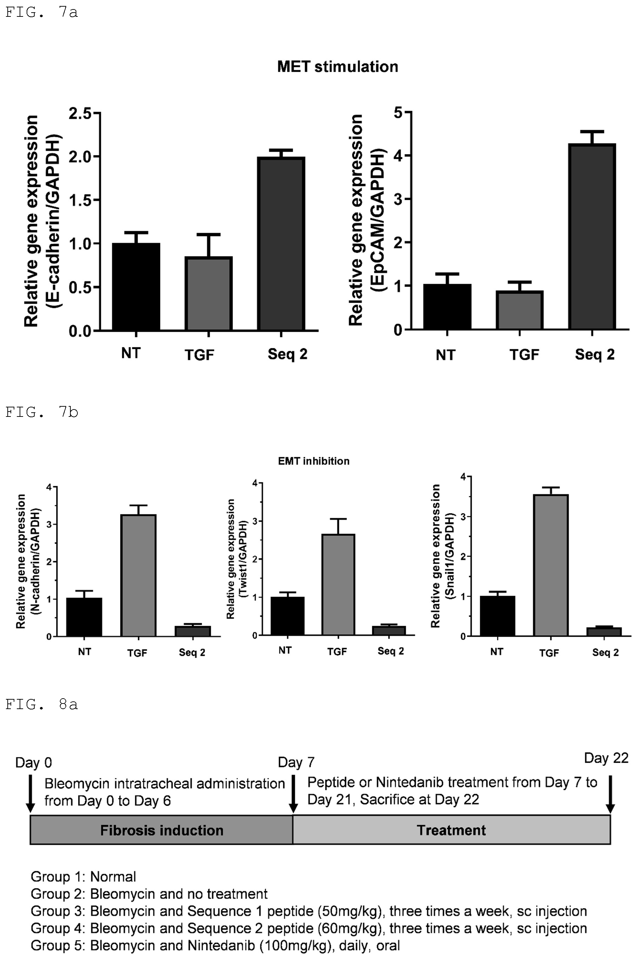

As a result, the peptide of SEQ ID NO: 2 increased the gene expression of E-cadherin and EpCAM, which are MET markers, and decreased the gene expression of N-cadherin, Twist1, and Snail1, which are EMT markers. Conversely, TGF-β1 decreased the expression of MET markers and increased the expression of EMT markers (FIG. 7). Accordingly, it was found that the peptide of SEQ ID NO: 2 increased MET and inhibited EMT.

### Example 7: Confirmation of therapeutic effect in animal model with bleomycin-induced lung disease

### 7-1: Animal testing method

In order to confirm the fibrosis therapeutic effect of the peptide according to the present invention at the animal level, an animal model in which lung disease was induced with bleomycin was used. C57BL/6J mice (male, 7 weeks old) were purchased from Charles River. Mice were anesthetized by intraperitoneal administration of a mixture of Imalgene (ketamine, 100 mg/kg)/Rompun (xylazine, 20 mg/kg), and a total of 50 µl of bleomycin sulfate (1.45 mg/kg, MBcell, CA, USA) was injected intratracheally once, and saline was injected intratracheally in the normal group (day 0). On day 7, mice were divided into groups, each consisting of eight. PBS (NT), SEQ ID NO: 1 (30 mg/kg), and SEQ ID NO: 2 (30 mg/kg) were administered subcutaneously three times a week, and nintedanib (Fluorochem Ref = 050741, 100 mg/kg) was administered orally daily. Each of the peptides of SEQ ID NO: 1 and SEQ ID NO: 2 was administered after dissolution in water for injection, and nintedanib was administered after mixing with water containing 0.5% hydroxyethylcellulose (Sigma; lot BCBV5033) and 0.01% Tween 80 (Sigma; lot BCBR6211V). While administering the drug for 21 days, body weight was measured every 2 days, and whether there were other clinical symptoms were checked. The experimental protocol is shown in FIG. 8a.

### 7-2: Observation of change in body weight

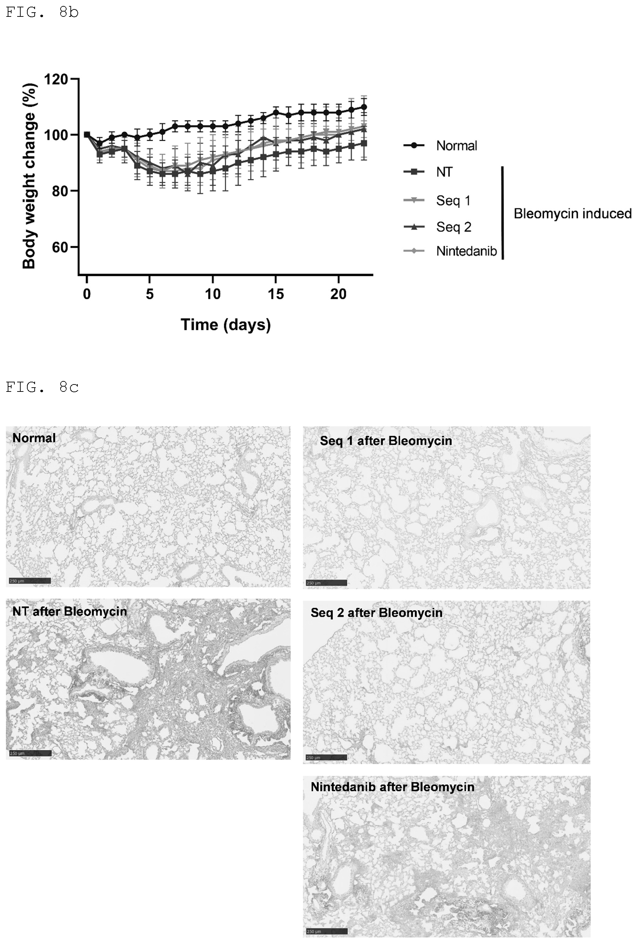

Based on results of measuring the change in body weight from day 0 to day 22, the body weight decreased until day 7 after administration of bleomycin, but increased after treatment with the peptide according to the present invention and nintedanib (FIG. 8b), and weight loss due to toxicity of the peptide according to the present invention and nintedanib was not observed.

### 7-3: Observation of fibrous deposition

After mice were sacrificed with isoflurane on day 22, lung tissue was excised therefrom and histological analysis was performed. Paraffin was introduced into the lungs, 5 µm paraffin sections were manufactured, Sirius Red staining and hematoxylin/phloxine staining were performed to evaluate the extent of pulmonary fibrosis and inflammation, and the extent of inflammation and fibrosis was observed through microscopy. Thereby, in the group (NT) which induced lung disease with bleomycin and to which PBS was administered, a lot of fibrous deposition was observed in the lungs, and in the nintedanib-administered group, there was some therapeutic effect, but a lot of deposited fibrous tissue was still observed. However, the group administered with the peptide of SEQ ID NO: 1 and the group administered with the peptide of SEQ ID NO: 2 were confirmed to significantly reduce fibrosis compared to the NT group and the nintedanib-administered group (FIG. 8c).

### 7-4: Measurement of area of fibrosis

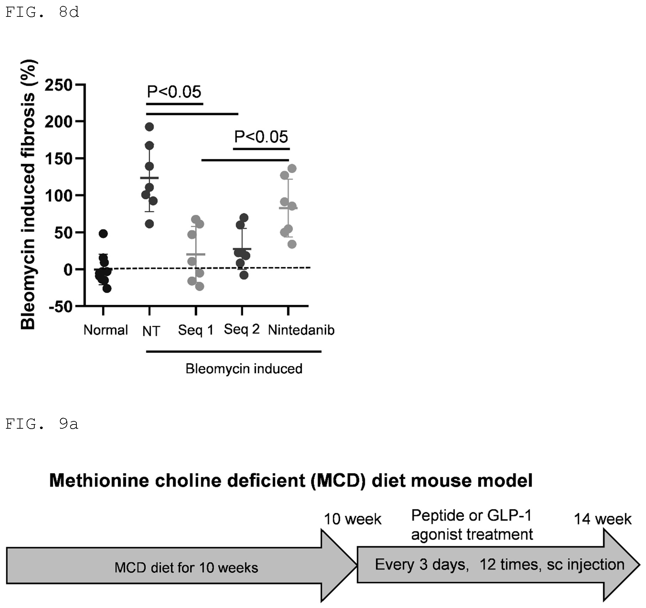

In order to quantify the fibrotic change, the area of the red (fibrotic portion) was quantified in the sample stained with Sirius Red. As a result, the groups treated with the peptide of SEQ ID NO: 1 and the peptide of SEQ ID NO: 2 showed a significant reduction in the area of fibrosis compared to the NT group and the nintedanib-treated group (FIG. 8d, P<0.05).

### Example 8: Confirmation of therapeutic effect in methionine-choline-deficient diet mouse model

### 8-1: Animal testing method

In order to further confirm the fibrosis therapeutic effect of the peptide according to the present invention at the animal level, hepatitis and hepatic fibrosis were induced by providing methionine-choline-deficient (MCD) diet to mice (methionine-choline-deficient diet mouse model). Male 6-week-old C57BL/6J mice were used, and divided into a normal diet group, a group not treated after feeding MCD diet, groups injected with the peptide of SEQ ID NO: 1 (20 mg/kg) and the peptide of SEQ ID NO: 2 (20 mg/kg), and a positive control group subcutaneously injected with liraglutide (0.2 mg/kg), which is a type of glucagon-like peptide 1 receptor agonist serving as a hepatitis therapeutic agent. After autonomous feeding of MCD diet for 10 weeks, 8 mice were assigned to each group and a therapeutic agent was administered thereto. Peptide and liraglutide were administered once a day, a total of 12 times at 3-day intervals (FIG. 9a).

### 8-2: Measurement of extent of hepatic inflammation

For all animals, body weight was measured immediately before initiation of administration and twice a week throughout the entire test. After the end of the last administration, the animals was anesthetized by intraperitoneal injection of 300 µl of Avertin, blood was collected from the abdominal vena cava by laparotomy, the liver and spleen were removed therefrom, and the weight of the removed organs was measured. Tissues were fixed in a neutral buffered formalin solution for all animals subjected to necropsy, and paraffin blocks were manufactured and sectioned through tissue processing. The tissue sections thus obtained were subjected to H&E staining. The H&E stained specimens were scored for steatosis, ballooning degeneration, and lobular inflammation (Table 3), and the extent of hepatic inflammation (NAFLD activity score) was measured.

**[Table 3] NAFLD activity score**

| Item | Definition | Score |
|---|---|---|
| Steatosis | < 5% | 0 |
| | 5-33% | 1 |
| | > 33-66% | 2 |
| | > 66% | 3 |
| Hepatocellular ballooning | None | 0 |
| | Few | 1 |
| | Many | 2 |
| Lobular inflammation | No foci | 0 |
| | < 2 foci per 200x field | 1 |
| | 2-4 foci per 200x field | 2 |
| | > 4 foci per 200x field | 3 |

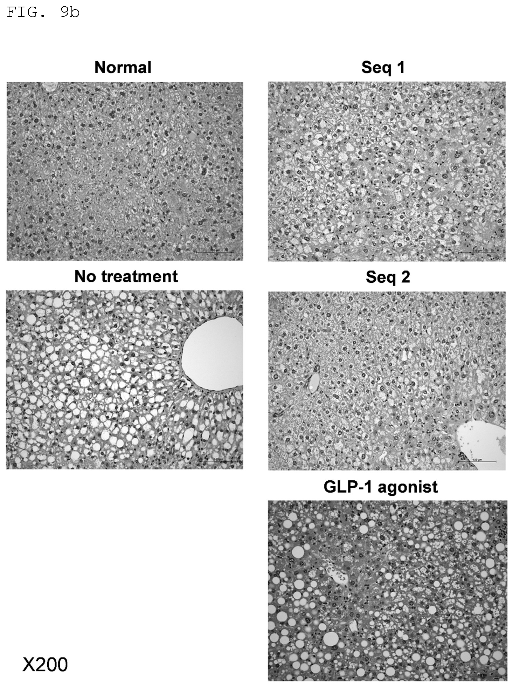

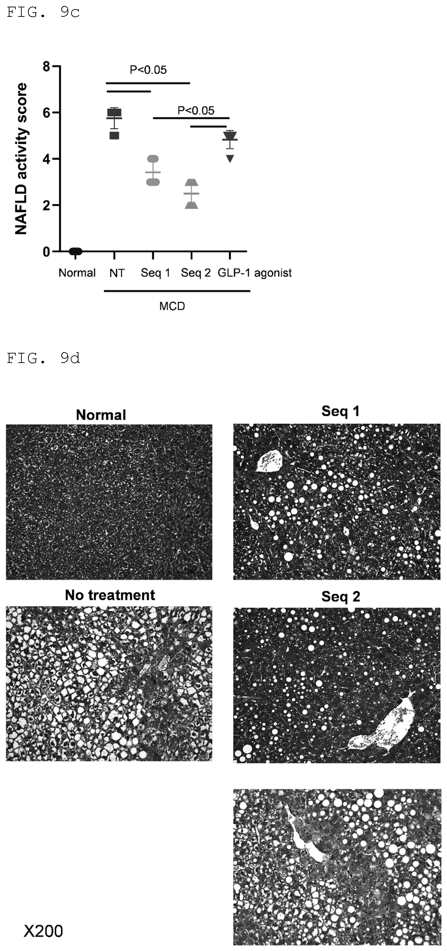

The results thereof are shown in FIGs. 9b and 9c. Both groups administered with the peptides of SEQ ID NO: 1 and SEQ ID NO: 2 significantly reduced the NAFLD activity score compared to the NT group or the positive control GLP-1 agonist-administered group.

### 8-3: Measurement of area of fibrosis

The tissue sections manufactured in the same manner as in Example 8-2 were stained with Masson's trichrome, and 100x magnification images were obtained with an optical microscope (BX51, Olympus, GER) at 5 randomly designated different sites among all Masson's trichrome-stained slides (300MI CMOS camera, Aptina). The blue-stained area was measured (Image-Pro Plus, Media Cybernetics Inc., UK) and analyzed as a percentage (Masson's trichrome-positive area (%)).

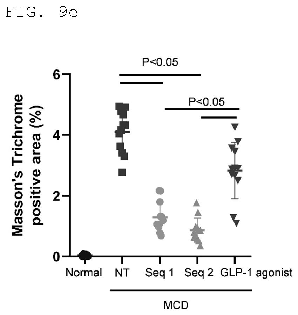

As a result, as shown in FIGs. 9d and 9e, both groups administered with the peptides of SEQ ID NO: 1 and SEQ ID NO: 2 were confirmed to significantly reduce the area of fibrosis compared to the NT group or the positive control GLP-1 agonist-administered group.

### Industrial Applicability

According to the present invention, a peptide represented by the amino acid sequence of SEQ ID NO: 1 and a peptide represented by the amino acid sequence of SEQ ID NO: 2 are capable of exhibiting superior therapeutic effects compared to nintedanib commercially available as a therapeutic agent for pulmonary fibrosis and liraglutide commercially available as a therapeutic agent for hepatic fibrosis, and thus can replace these commercially available therapeutic agents and can be used in combination with these therapeutic agents.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### Sequence List Free Text

An electronic file is attached.
The following clauses, describing aspects of the invention, are part of the description:
[Clause 1] A peptide for preventing or treating fibrosis represented by an amino acid sequence of SEQ ID NO: 1 or SEQ ID NO: 2.
[Clause 2] The peptide according to clause 1, wherein the peptide represented by the amino acid sequence of SEQ ID NO: 1 inhibits dissociation of TGF-β1 from a large latent complex (LLC) by binding to integrin.
[Clause 3] The peptide according to clause 1, wherein the peptide represented by the amino acid sequence of SEQ ID NO: 2 inhibits epithelial-mesenchymal transition, or binds to Smad2 and Smad3 through cell penetration to thus inhibit phosphorylation thereof.
[Clause 4] A pharmaceutical composition for preventing or treating fibrosis comprising a peptide represented by an amino acid sequence of SEQ ID NO: 1 and/or a peptide represented by an amino acid sequence of SEQ ID NO: 2 as an active ingredient.
[Clause 5] The pharmaceutical composition according to clause 4, wherein the fibrosis is selected from the group consisting of pulmonary fibrosis, hepatic fibrosis, renal fibrosis, and cardiac fibrosis.
[Clause 6] The pharmaceutical composition according to clause 4, wherein the pharmaceutical composition is formulated in any one dosage form selected from the group consisting of an injection, a formulation for oral administration, a patch, a liquid, a capsule, a granule, a tablet, a powder, a spray, a formulation for nasal administration, an inhalation spray, an ointment, a gel, a formulation for mucosal administration, and a suppository.
[Clause 7] The pharmaceutical composition according to clause 4, wherein the pharmaceutical composition further compries an acceptable adjuvant.
[Clause 8] The pharmaceutical composition according to clause 4, wherein the peptide is administered daily at 1 µg/kg to 100 mg/kg, and is administered every day or 1-3 times a week.

## Claims

1. A peptide for use in a method of preventing or treating fibrosis represented by the amino acid sequence of SEQ ID NO: 1, wherein the method comprises subcutaneous injection to a subject in need thereof.

2. The peptide for use according to claim 1, wherein the peptide represented by the amino acid sequence of SEQ ID NO: 1 inhibits the dissociation of TGF-β1 from the Large Latent Complex (LLC) by binding to integrin.

3. A pharmaceutical composition for use in a method of preventing or treating fibrosis comprising the peptide represented by the amino acid sequence of SEQ ID NO: 1 as an active ingredient, wherein the pharmaceutical composition is an injection.

4. The pharmaceutical composition for use according to claim 3, wherein the fibrosis is selected from the group consisting of pulmonary fibrosis, liver fibrosis, renal fibrosis and cardiac fibrosis.

5. The pharmaceutical composition for use according to claim 3, wherein the pharmaceutical composition further contains an acceptable adjuvant.

6. The pharmaceutical composition for use according to claim 3, wherein the peptide is administered daily.

7. The pharmaceutical composition for use according to claim 3, wherein the peptide is administered weekly.

8. The pharmaceutical composition for use according to claim 3, wherein the injection is a subcutaneous injection.

9. The pharmaceutical composition for use according to claim 6, wherein the peptide is administered at a dose of 100 mg daily.

10. The pharmaceutical composition for use according to claim 6, wherein the peptide is administered at a dose of 200 mg daily.

11. The pharmaceutical composition for use according to claim 6, wherein the peptide is administered at a dose of 300 mg daily.

12. The pharmaceutical composition for use according to claim 6, wherein the peptide is administered at a dose of 400 mg daily.

13. The pharmaceutical composition for use according to claim 7, wherein the peptide is administered at a dose of 400 mg weekly.
